# EUROPEAN PATENT APPLICATION

(11) **EP 4 652 966 A1**
(43) Date of publication of application: **26.11.2025**
(21) Application number: 25177757.9
(22) Date of filing: 20.05.2025
(51) Int. Cl.: A61F 2/30, A61F 2/38, A61F 2/46

(54) **KNEE REPLACEMENT IMPLANTS AND INSTRUMENTS**

(30) Priority: 20.05.2024 US 202418407582
(71) Applicant: Globus Medical, Inc., Audubon, PA 19403 (US)
(72) Inventor: ZAPPACOSTA, Jason, PHILADELPHIA, 19130 (US); STUMPO, David, TRAPPE, 19426 (US); CONLEY, Ryan, WEST CHESTER, 19380 (US)
(74) Representative: Morabito, Sara

(57) **Abstract**

Knee arthroplasty implants, instruments, systems, and methods of implanting the same. The knee arthroplasty implants may include a femoral implant (10) and a tibial tray implant (110), each with trabecular surfaces (44, 144) configured to contact bone to provide a scaffold for bone healing and interdigitation. The implants may also include fixation pegs (60, 160) with bulleted profiles to increase the surface area for bone ingrowth. An inserter instrument (200) may include a sliding rack (234), which is incrementally extended with a rotatable shaft (216), thereby allowing for quick lock and release of various tibia trays.

## Description

### FIELD OF THE INVENTION

The present application relates generally to knee arthroplasty, and more particularly, to knee arthroplasty implants, instruments, and methods of installing knee arthroplasty implants.

### BACKGROUND OF THE INVENTION

Knee arthroplasty, often called a knee replacement, is a surgical procedure used to reconstruct and resurface a knee that has been damaged, for example, by arthritis. Total knee arthroplasty (TKA) devices may replace both the tibiofemoral joint and the patellofemoral joint. The tibiofemoral joint is where the tibia and the femur articulate. The patellofemoral joint is where the patella and the femur articulate. To replace the tibiofemoral joint, the knee arthroplasty may include a femoral implant secured to the distal end of the femur (or thigh bone), a tibial tray implant secured to the proximal end of the tibia (or shin bone), an insert disposed therebetween, and an optional patella backing. The femoral and tibial implants cap the ends of the femur and tibia, respectively, which form the knee joint, thereby reconstructing the knee. To replace the patella-femoral joint, the knee arthroplasty may include a patella prostheses (or implant) to replace the backside of the patella and form a replacement articulating surface which interfaces with the femoral implant.

The femoral portion of the procedure may include removing damaged bone and cartilage at the end of the femur and cutting the resurfaced end of the bone to fit the femoral implant. The tibial portion of the procedure may include a planar resection to the proximal tibia, which is replaced with the tibial tray implant. In some cases, the implants may be adhered to bone using bone cement. The implants may include recesses for interdigitation of the cement. There are risks, however, associated with the bone cement. For example, the bone cement may fail by not producing a strong enough bond or may weaken over time causing the knee replacement to fail, cement fracture, or insufficient cement application. These failures can result in complications such as inflammation, swelling, and persistent pain. Additionally, cement carries its own risks and surgical challenges, such as cement impingement, improper mixing techniques, and proper timing of the cement mixing and application. There exists a need for improved femoral implants and tibial trays that may be implanted without cement, and improved instruments configured to implant the components.

### SUMMARY OF THE INVENTION

To meet this and other needs, knee arthroplasty implants, instruments, systems, and methods of installing the same are provided. In particular, the knee arthroplasty implants may include a femoral implant and a tibial tray implant, each with trabecular surfaces configured to contact bone to provide a scaffold for bone healing and interdigitation. The implants may also include fixation pegs with bulleted profiles to provide initial fixation and increase the surface area for bone ingrowth. Specialized instrumentation may be particularly suited for installing the implants.

According to one embodiment, a system for a knee arthroplasty includes a femoral implant and a tibial tray implant. The femoral implant has an anterior flange, a pair of posterior condylar flanges, and a distal portion therebetween. The femoral implant has an outer articulating surface with a smooth rounded shape that closely approximates an exterior distal femur surface of a natural human knee, an inner surface shaped to match a resected femur with five resection cuts, and one or more pegs extending from the inner surface. The tibial tray implant has a plate with a perimeter wall defining an insert receiving space, a distal surface configured to contact a resected tibia, a keel extending from the distal surface, and one or more pegs extending from the distal surface. The inner surface of the femoral implant and the distal surface of the tibial tray define a trabecular structure, which provides a scaffold for bone healing and interdigitation.

The knee arthroplasty system may include one or more of the following features. The trabecular structure may have a grid, lattice, or honeycomb pattern to promote bony in-growth. The trabecular structure may haves a porosity in the range of 50-80%. A thickness of the trabecular structure may be oversized for an increased press fit in trabecular structure areas to ensure contact with the resected femur and tibia. The trabecular structure may be created by three-dimensional printing. The pegs of the femoral implant and tibial tray implant may each have a bullet shaped profile with six fins radiating outward from the peg. The inner surface of the femoral implant may include an anterior femur cut surface, a posterior femur cut surface, a distal femur cut surface, an anterior chamfer cut surface, and a posterior chamfer cut surface. The trabecular structure may extend along the anterior femur cut surface, the posterior femur cut surface, the distal femur cut surface, the anterior chamfer cut surface, and the posterior chamfer cut surface. The trabecular structure may be surrounded by a solid wall. The femoral implant and tibial implant may be cementless such that they are securable to bone without cement.

According to one embodiment, a system for a knee arthroplasty includes a tibial tray and an inserter. The tibial tray has a perimeter wall defining an insert receiving space. The perimeter wall defines a pair of posterior notches and a single anterior notch. The inserter has a main body with foot having a pair of fixed posterior tabs, a sliding rack having an anterior tab, and a rotatable shaft for controlling movement of the sliding rack. The posterior tabs are receivable in the posterior notches of the tibial tray and the anterior tab is receivable in the anterior notch of the tibial tray when the shaft is rotated and the sliding rack is translated into an extended position.

The knee arthroplasty system may include one or more of the following features. The rotatable shaft of the inserter may have a gear wheel with a gear face at its distal end. The sliding rack may have a linear gear track with teeth that intermesh with the gear face of the rotatable shaft. The foot may be bifurcated by a keyway, and the sliding rack may be receivable in the keyway. The tibial tray may have a kidney-bean shape with an anterior side forming an outer convex side and a posterior side including an inner concave side separating two lobes, and the foot may have an outer kidney-bean shape matching the kidney-bean shape of the tibial tray. The tibial tray may include a keel attached to a distal surface of the tibial tray, and the keel may include a pair of coronal fins and a sagittal fin.

According to one embodiment, a method for implanting a tibial implant may include one or more of the following steps in any suitable order: (1) positioning fixed posterior tabs on a main body of an inserter into corresponding posterior notches in a perimeter wall of a tibial tray; (2) tilting the inserter and/or tibial tray until a foot of the main body of the inserter is received in an insert receiving space of the tibial tray; (3) rotating a shaft through the main body of the inserter to translate a sliding rack having an anterior tab and extend the anterior tab into an anterior notch in the tibial tray, thereby locking the inserter to the tibial tray; (4) impacting an impaction cap to seat the tibial tray into a proximal tibia of a patient; (5) removing the inserter by rotating the shaft in the opposite direction and withdrawing the sliding rack from the tibial tray; and (6) attaching an insert with tabs receivable in the posterior and anterior notches in the tibial tray. The method may also include, before positioning the inserter, resecting the proximal tibial to form a planar resection surface, and punching a cavity into the proximal tibia, the cavity being configured to receive a keel of the tibial tray.

Also provided are kits including implants of varying types and sizes including femoral implants, tibial trays, and inserts that vary in anterior-posterior (AP) and/or medial-lateral (ML) aspects, instruments of varying types and configurations including inserter instruments, and other components for performing the procedure.

### BRIEF DESCRIPTION OF THE DRAWINGS

A more complete understanding of the present invention, and the attendant advantages and features thereof, will be more readily understood by reference to the following detailed description when considered in conjunction with the accompanying drawings, wherein:
FIG. 1 shows a perspective view of a femoral implant showing the inner trabecular structure according to one embodiment;
FIGS. 2A-2B show a side view and top view, respectively, of the femoral implant of FIG. 1;
FIGS. 3A-3B show a side view and close-up view, respectively, of the fixation peg having a bulleted profile and fins according to one embodiment;
FIG. 4 shows a perspective view of a tibial tray implant according to one embodiment;
FIG. 5 shows a bottom view of the tibial tray implant of FIG. 4 showing the trabecular structure according to one embodiment;
FIGS. 6A-6B show close-up views of the bottom of the tibial tray implant with and without the trabecular structure, respectively;
FIGS. 7A-7B show perspective views of an inserter instrument for installing the tibial tray implant according to one embodiment;
FIG. 8 shows an exploded view of the inserter instrument; and
FIGS. 9A-9B show front and side views, respectively, of the inserter instrument mated with the tibial tray implant for implantation according to one embodiment.

### DETAILED DESCRIPTION OF THE INVENTION

Embodiments of the disclosure are generally directed to implants, instruments, systems, and methods for implanting knee replacement implants including a femoral implant and a tibial tray implant, for example, during a total knee arthroplasty. Total knee arthroplasty systems may include a femoral component, a tibial component, and an insert positioned between the femoral and tibial components. The femoral component may be secured to the femur after resurfacing and resecting the end of the bone to fit the femoral implant. The tibial tray implant may be secured to the tibia after a planar resection to the proximal tibia. Specifically, embodiments are directed to cementless femoral implants and tibial tray implants, each having a trabecular structure, which interfaces with the bone. The trabecular structure may include a porous scaffold, which is configured for bone to grow into the structure. As the bone heals, the bone grows into the microscopic porous structure further enhancing fixation of the implants.

The implant assembly may be comprised of one or more biocompatible materials. For example, the femoral and tibial implants may be made from a metal, such as titanium, stainless steel, cobalt chrome, carbon composite, or suitable alloys. The insert may be made from a plastic or polymer, such as polyethylene, ultra-high molecular weight polyethylene (UHMWPE), polyetheretherketone (PEEK), or combinations of such materials. In this manner, the components are configured so that metal articulates against plastic in order to provide smooth movement and minimal wear. These materials may be machined, constructed from additive manufacturing, such as three-dimensional (3D) printing, subtractive manufacturing, or hybrid manufacturing processes. Although the materials described herein are exemplified, it will be appreciated that any suitable materials and construction may be selected for the individual components.

Although generally described with reference to a knee arthroplasty, it will be appreciated that the implants, instruments, and systems described herein may be applied to other orthopedic locations and applications, such as the spine including between vertebrae, long bones, such as a femur, a tibia, a humerus, a clavicle, a fibula, an ulna, a radius, bones of the foot, bones of the hand, or other suitable bone(s) or joints.

Additional aspects, advantages and/or other features of example embodiments of the invention will become apparent in view of the following detailed description. It should be apparent to those skilled in the art that the described embodiments provided herein are merely exemplary and illustrative and not limiting. Numerous embodiments and modifications thereof are contemplated as falling within the scope of this disclosure and equivalents thereto.

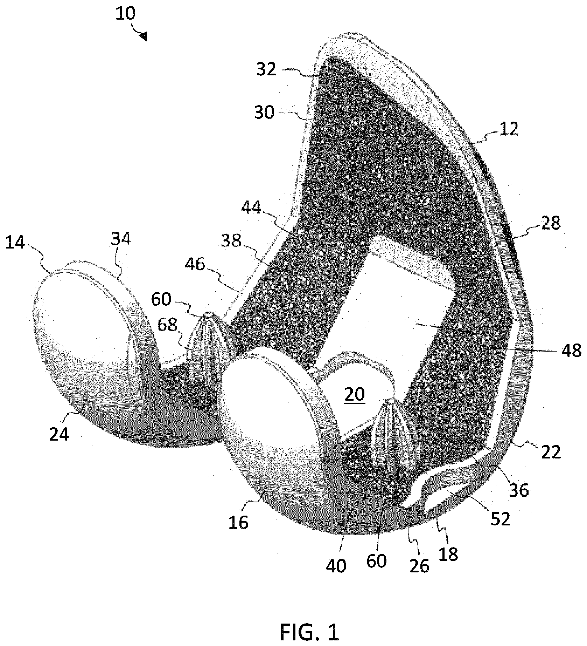

Turning now to the drawing, where like reference numerals may refer to like elements, FIGS. 1-2B shows a femoral implant 10 according to one embodiment. The femoral implant 10 replaces the end of the femur or thigh bone, for example, after a resection. The implant 10 curves over the front and back of the distal end of the femur and recreates the natural shape of an original femur for proper knee function. In this case, the femoral implant 10 is configured to be secured to the bone without cement, thereby eliminating the need for cement and the risks associated therewith.

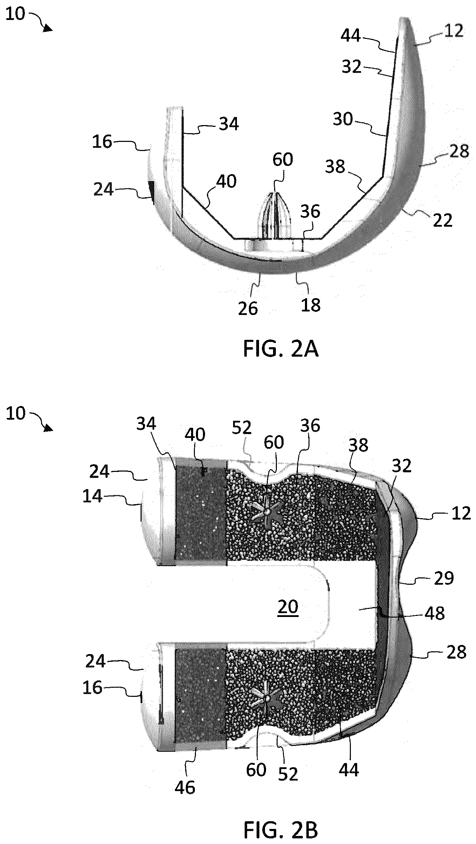

The femoral implant 10 includes an anterior flange 12, a pair of posterior condylar flanges 14, 16, and a distal portion 18 therebetween. The pair of posterior condylar flanges 14, 16 include a medial condyle portion 14 and lateral condyle portion 16 configured to mimic the condyles of a natural femur. The medial and lateral condyle portions 14, 16 are spaced apart from one another (e.g., generally along the medial-lateral direction) defining an intercondylar notch 20 therebetween. As best seen in FIG. 2B, the intercondylar notch 20 may be generally rectangular in shape with rounded corners, or a rounded rectangle. In the case of a posterior stabilized implant, the intercondylar notch 20 may form a box that accepts a stabilizing post or cam. For the posterior stabilized implant, the intercondylar box 20 may help to stabilize the posterior translation of the tibia. In some instances, the intercondylar box 20 may include additional material for migration resistance.

An exterior or outer articulating surface 22 of the implant 10 may form a rounded C-shape corresponding to the natural distal femoral surface of a human knee. The outer articulating surface 22 may include outer condylar surfaces 24 and outer distal surface 26 for cooperation with the corresponding end of a tibia and outer anterior surface 28 for cooperation with the patella. As best seen in FIG. 2B, the outer anterior surface 28 may be separated by a trochlear groove 29 that closely approximates the anterior distal femur surface of a natural human knee.

The femoral implant 10 may be configured to be secured to a resected femur. For example, the femur component 10 may have an inner or interior surface 30 that is shaped to match with five femur resection cuts. The 5-cut bone interfacing surface 30 may include an anterior femur cut surface 32, a posterior femur cut surface 34, a distal femur cut surface 36, an anterior chamfer cut surface 38, and a posterior chamfer cut surface 40. The distal femur cut surface 36 mates with a resection cut to the distal-most femoral condyle. The distal resection cut may influence the mechanical alignment, extension gap, and joint line height. The anterior femur cut surface 32 mates with an anterior resection cut to the distal femur, which is cut through the trochlear groove. The depth of the anterior resection cut may affect the patellofemoral joint. The posterior femur cut surface 34 mates with a posterior resection cut to the distal femur, which is cut through the posterior femoral condyles. The posterior resection cut may affect the flexion gap. The anterior and posterior cuts may be generally parallel and may determine the rotation of the femoral implant. The anterior flange cut 32 and posterior flange cut 34 may have a taper, such as a 6-degree taper, for a press fit on the resected femur. The anterior chamfer cut surface 38 is an angled cut, which connects the anterior cut surface 32 and the distal surface 36 and the posterior chamfer cut surface 40 is an angled cut, which connects the posterior cut surface 34 and the distal surface 36. These chamfer cuts 38, 40 also mate with corresponding resection cuts on the distal end of the femur. Before making the resection cuts, the implant size may be selected to determine the desired resection cut locations to ensure optimal fit with the interior surface 30 of the implant 10.

The interior surface 30 defines a trabecular structure 44, which provides a scaffold for bone healing and bone interdigitation. The trabecular structure 44 replaces the need for cement because as the bone heals, the bone grows into the microporous structure further enhancing fixation. The trabecular structure 44 may include a porous scaffold structure, for example, including hexagonal struts with micropores. In some embodiments, the trabecular structure 44 may have a grid, lattice, or honeycomb pattern to promote bony in-growth. The trabecular structure 44 may include a randomized or repeating pattern of open or interconnected pores. The pores may be spherical, partially spherical, or of another suitable pore shape or configuration. The trabecular structure 44 may have a suitable porosity (open volume), for example, greater than 50% open, greater than 60% open, greater than 70% open. In one embodiment, the trabecular structure 44 may have a porosity in the range of about 50-80% to maximize the potential for bony in-growth. The trabecular structure 44 may have pore sizes, for example, ranging from approximately 100µm-2mm, approximately 100µm-1mm, approximately 200-900µm, or approximately 300-800µm in diameter. Additional details on suitable porous structures are described, for example, in U.S. Patent No. 10,524,926, which is incorporated by reference herein in its entirety for all purposes.

The trabecular structure 44 may be created by additive manufacturing, such as three-dimensional (3D) printing, while printing the entire component. The additive manufacturing may include direct metal laser sintering (DMLS), powder bed fusion, vat photopolymerization, material jetting, lamination, extrusion, directed energy deposition, or any other suitable additive manufacturing process. The implants 10 may also be manufactured utilizing a combination of additive manufacturing processes and other manufacturing processes, such as laser etching, abrasion, machining, polishing, and chemical treatment. The implant 10 may be created completely via an additive process or may include a hybrid build by machining a base and then adding the trabecular structure 44 via additive manufacturing.

The trabecular structure 44 may be provided along the interior surface 30 of the implant 10 including the anterior femur cut surface 32, the posterior femur cut surface 34, the distal femur cut surface 36, the anterior chamfer cut surface 38, and the posterior chamfer cut surface 40. The trabecular structure 44 may be surrounded by a solid edge, rim, or wall 46. In other words, the outer perimeter of the implant 10 may form a solid wall 46 and the inner area may be filled with the trabecular structure 44. The thickness of the trabecular structure 44 may be oversized for an increased press fit in the trabecular structure areas. As best seen in FIG. 2A, the trabecular structure 44 may protrude past the wall 46 of the implant 10 to have increased contact with the resected cuts. This ensures the trabecular structure 44 is always in contact with the resected femur bone.

A central portion 48 of the implant 10 may have a solid or smooth area absent of the trabecular structure 44. The central portion 48 may extend from the notch 20, along the anterior chamfer 38, and into a small area of the anterior cut surface 32. The central portion 48 may include a rectangular area with rounded corners or another suitable shape to support the implant 10. The solid central portion 48 may add extra structure to the center of the implant 10, thereby providing additional structural integrity and mechanical stability while maximizing the area of the trabecular structure 44 to facilitate better integration/incorporation with the adjacent bone.

The edges or walls 46 of the implant 10 may define one or more recesses 52, for example, configured to interface with an insertion instrument. Each recess 52 may define a curved indentation for receiving a complementary portion of the instrument. The curved recess 52 may have, for example, a concave semi-circular shape. A pair of recesses 52 may be located along the outside wall 46 on opposite sides of the distal femur cut 36.

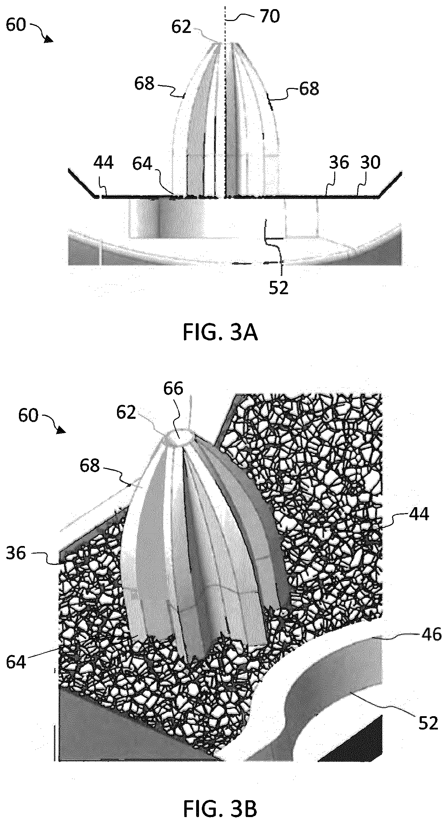

In addition to the 5-cut bone interfacing surface 30, the implant 10 may include one or more, anchoring projections, fixation spikes, or pegs 60, which may be press fit into the resected distal surface of the femur. As best seen in the close-up views in FIGS. 3A-3B, each fixation peg 60 may include a bulleted profile extending vertically from the distal femur cut surface 36. The bulleted profile may include a pointed or rounded tip 62 that tapers to a wider base 64. The pegs 60 may be solid or hollow (e.g., have a solid or hollow core). In one embodiment, the peg tip 62 may include a curved or semi-spherical indentation 66, for example, forming a hollow-point (negative cone) end. Each fixation peg 60 may have one or more peripheral ribs or fins 68 arranged about a central fin axis 70. The fins 68 may have rounded, chamfered, sharpened, or fillet edges. The fins 68 may include radial projections extending outward around the circumference of the peg 60. The fins 68 may radiate outward between the narrowed tip 62 and wider base 64 of the peg 60. The fins 68 may be evenly spaced about the peg 60 or otherwise configured. The fins 68 may be configured to minimize bone displacement, minimize the risk of fracture, and/or increase the surface area for bone ingrowth. In one embodiment, the fixation peg 60 has a six-sided fin design, although it will be appreciated that any suitable number of fins 68 may be selected. The implant 10 may include a pair of fixation pegs 60 located on the distal femur cut surface 36, although it will be appreciated that any suitable number and location of pegs 60 may be selected to secure the implant 10 to bone. The fixation pegs 60 are configured to provide increased surface area, which is conducive for bone interdigitation and enhanced fixation to the bone.

The femoral implant 10 may be secured to the femur via the 5-cut bone interfacing surface 30, the fixation pegs 60, and the trabecular structure 44 to enhance initial and long-term fixation. First, the implant 10 is secured to the femur by nature of the interfacing surface 30, which may be press fit onto the resected femur. Initial fixation is also achieved by the press fit of the fixation pegs 60 into the resected distal surface. The fins 68 also provide greater surface area for bone interdigitation over time. In the case of a posterior stabilized implant, box 20 may add further strength for migration resistance. Lastly, long term stability may be provided through the trabecular structure 44, which provides a scaffold for bone healing and interdigitation over time. By providing a cementless implant 10, the implant 10 may be installed without cement, which eliminates cement related complications. In addition, a cementless procedure may result in reduced procedural times and decreased surgeon stress.

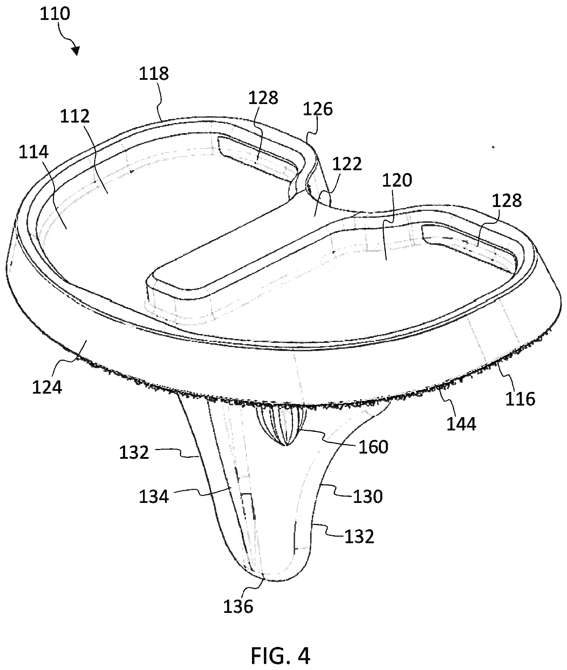

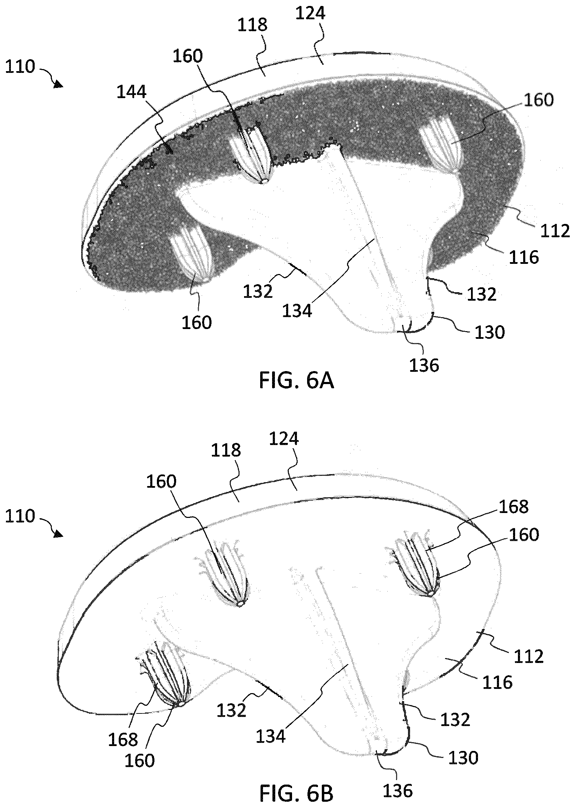

Turning now to FIGS. 4-6B, a tibial tray implant 110 is shown according to one embodiment. The tibial tray implant 110 replaces the end of the tibia or shin bone, for example, after a resection. Similar to femoral implant 10, tibial tray implant 110 include a trabecular structure 144 configured to be secured to the bone without cement, thereby eliminating the need for cement and the risks associated therewith. The tibial tray 110 may be initially secured to bone with one or more keels 130 and pegs 160 to prevent any movement of the tibial tray 110. Sustained stability may be achieved through the trabecular structure 144, which serves as a scaffold that supports and promotes bone regeneration over time.

The tibial tray 110 may be offered in multiple sizes that vary in the anterior-posterior (AP) and medial-lateral (ML) aspects in order to conform to patient anatomy. The tray 110 may include a keel structure 130 on the distal surface 116 of the tray 110, as well as one or more pegs 160, which offer additional support/fixation to the cancellous bone of the tibia. A keel punch may be utilized to create an initial cavity within the cancellous bone of the tibia to allow for the keel 130 of the tibial tray 110 to seat into the proximal tibia. One or more additional openings may be created in the resection to allow for receipt of the pegs 160. After all required preparation of the tibia has taken place, the tibial tray 110 is implanted into the resected and punched proximal tibia. The tibial tray 110 may be impacted into place to ensure proper seating of the tray 110 has occurred. After the tray 110 is installed and inserter removed therefrom, an insert (not shown) may be connected to the tray 110 to complete the tibial implant assembly. Similar to implant 10, the trabecular structure 144 provides a microporous structure for the bone to grow into, thereby providing long term stability.

The tibial tray 110 includes a plate 112 having opposite proximal and distal surfaces 114, 116. The distal surface 116 of the tibial plate 112 is configured to engage the resected surface of the tibia. The tibial plate 112 has a perimeter edge, wall, or lip 118 defining an insert receiving space 120 sized and shaped to receive the insert (not shown). The proximal surface 114 of the plate 112 defines the distal or bottom surface of the insert receiving space 120. The insert receiving space 120 may be divided into two areas by a raised portion or solid center strut 122, which may provide increased fatigue endurance.

The tibial plate 112 includes an anterior side 124 and an opposite posterior side 126. The outer profile of the tibial tray 110 may be rounded or curved. For example, the tibial tray 110 may have the general shape of a long oval indented at one side, such as a kidney-bean shape. The anterior side 124 may form an outer convex side as one long side and the posterior side 126 may include an inner concave side separating two lobes or rounded ends. The center strut 122 may extend from the central concavity on the posterior side 126, a distance toward the anterior side 124, while leaving a gap between the two open areas of the insert receiving space 120. The perimeter wall 118 of the tray 110 may define one or more recesses, pockets, or notches 128 used to receive a portion of the insert to hold the insert in the insert receiving space 120 of the tibial tray 110. For example, a pair of posterior notches 128 may be provided along the posterior side 126 of the wall 118 and a single anterior notch 128 (not visible) may be provided along the anterior side 126 of the wall 118 for interfacing with the insert and/or an insertion instrument, such as instrument 200.

The insert (not shown) sits between the tibial tray 110 and the femoral implant 10 to mimic the motion of a natural knee and provide support as the knee is bent and flexed. The insert may fit inside receiving space 120 to provide an articulating or articular surface configured to interface with the femoral implant 10. Additional details on inserts and tibial tray implants are provided in U.S. Patent Publication No. 2023/0270563, which is incorporated by reference herein in its entirety for all purposes.

The distal surface 116 of the plate 112 defines trabecular structure 144, which provides a scaffold for bone healing and bone interdigitation. The trabecular structure 144 replaces the need for cement because as the bone heals, the bone grows into the microporous structure further enhancing fixation. In one embodiment, the entire bottom surface 116 of the implant 110 includes trabecular structure 144 to facilitate bone growth. The trabecular structure 144 may include a porous scaffold structure, for example, including hexagonal struts with micropores. In some embodiments, the trabecular structure 144 may have a grid, lattice, or honeycomb pattern to promote bony in-growth. The trabecular structure 144 may include a randomized or repeating pattern of open or interconnected pores. The pores may be spherical, partially spherical, or of another suitable pore shape or configuration. The trabecular structure 144 may have a suitable porosity (open volume), for example, greater than 50% open, greater than 60% open, greater than 70% open. In one embodiment, the distal face 116 may have a porosity in the range of about 50-80%. The trabecular structure 144 may have pore sizes, for example, ranging from approximately 100µm-2mm, approximately 100µm-1mm, approximately 200-900µm, or approximately 300-800µm in diameter.

The trabecular structure 144 may be created by additive manufacturing, such as three-dimensional (3D) printing, while printing the entire component. The implant 10 may be manufactured in a complete additive manufacturing process where all of the implant geometry is built upon a base plate, post processed, and removed from the base plate. Alternatively, the implant 10 may be manufactured in a hybrid build where the lattice portion of the implant is created either by machining from a blank plate or from a forged blank. Then, the blank is put into the additive machine and both lattice and solid geometry is built upon the blank. By building the part using additive manufacturing, the highly porous lattice structure 144 can be built, which may not be otherwise created using traditional manufacturing methods.

The trabecular structure 144 may form the distal face 116 of the tray 110. The thickness of the trabecular structure 144 may be oversized, for example, having a thickness ranging from about 1 to 1.5mm. As best seen in FIG. 4, the trabecular structure 144 may protrude past the plate 112 such that the trabecular structure 144 has increased contact with the resected cut. This ensures the trabecular structure 144 is in optimal contact with the resected femur bone to promote bony in-growth.

The tibial implant 110 may include a tibial stem or keel 130. The tibial keel 130 is configured to be inserted into the punched cavity in the resected surface at the proximal end of the tibia of the patient. The tibial keel 130 is attached to the distal surface 116 of the tibial plate 112. The tibial keel 130 extends generally distally from the distal surface 116 of the tibial plate 112. The tibial keel 130 may be solid or hollow (e.g., having a solid or hollow core).

The tibial keel 130 may include one or more fins 132, 134 including coronal fins 132 and/or sagittal fins 134. The coronal fins 132 (e.g., two coronal fins 132) may extend outward from the center of the tibial keel 130 in a direction that is generally parallel to a coronal plane of the patient (e.g., a vertical side-to-side extending plane). The coronal fins 132 may be provided at a slight angle relative to the coronal plane, for example, about 15 degrees or less to form a slight V-shape. The sagittal fin 134 (e.g., single sagittal fin 134) may extend outward from the center of the tibial keel 130 in a direction that is generally parallel to a sagittal plane of the patient (e.g., a vertical front-to-rear extending plane). The coronal and sagittal fins 132, 134 may taper inwardly or narrow as the fins 132, 134 extend distally. The width of the sagittal fin 134 may also taper inwardly (e.g., in a direction generally parallel to the coronal plane) as the fin 134 extends distally. The fins 132, 134 may have rounded edges to improve insertion into the cavity. The distal-most nose or tip 136 of the tibial keel 130 may be tapered or curved, for example, along the coronal plane and/or the sagittal plane. The nose 136 is shown curved in the coronal plane but it may be blunt or tapered in both the coronal and sagittal planes. It will be appreciated that other configurations or modifications to the tibial keel 130 may be provided to enhance insertion and retention within cavity in the proximal tibia.

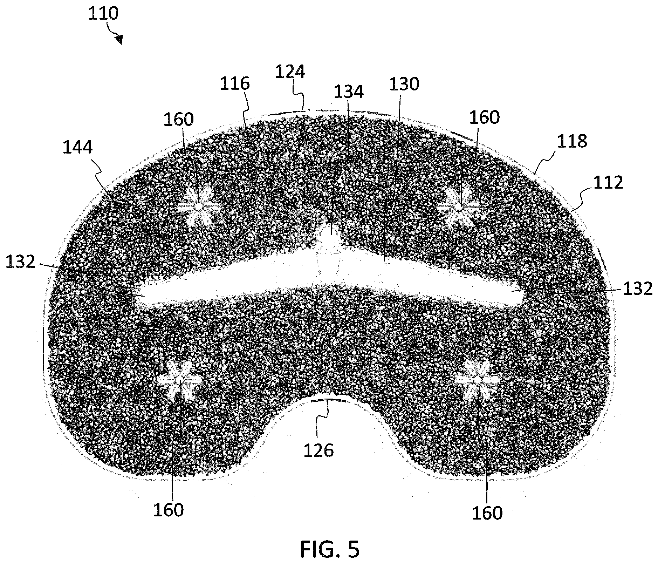

Similar to implant 10, the tibial tray 110 may further include one or more anchoring projections, fixation spikes, or pegs 160. Each peg 160 is configured to be inserted into corresponding openings in the resection of the proximal tibia of the patient to aid resistance to movement. The pegs 160 may be provided on the tray 110 in a cementless procedure to provide addition support and/or fixation to the cancellous bone of the tibia. The pegs 1602 extend generally distally from the distal surface 116 of the tibial plate 112. Similar to pegs 60, the pegs 160 may have a generally bulleted shape or other suitable shapes may be used. The pegs 160 may include one or more peripheral ribs or fins 168, for example, with rounded, chamfered, sharpened, or fillet edges. The fins 168 may be configured to minimize bone displacement, the risk of fracture, and/or increase the surface area for bone ingrowth. The pegs 160 may be spaced apart from one another about the distal surface 116 of the tibial plate 112. For example, as best seen in FIG. 5, the pegs 160 may be arranged around the tibial keel 130 with the tibial keel 130 positioned centrally between the pegs 160. For example, two pegs 160 may be located toward the anterior side 124 and two pegs 160 may be located toward the posterior side 126 of the implant 110. Any suitable quantity and arrangement of the pegs 160 may be provided to achieve the desired support and fixation to the tibial tray 110. The pegs 160 may be generally identical to one another or may be otherwise configured.

As shown in FIG. 6B with the trabecular structure 144 omitted for clarity, the keel 130 and pegs 160 may extend from base plate 112. The trabecular lattice structure 144 forms a border around the base of the keel 130 and pegs 160 and fills in the entire distal face 116 of the plate 112. The implant 110 may be created by a pure additive build where the entire implant 110 is created in a 3D manufacturing method. Alternatively, the implant 110 may be created using a hybrid build where the trabecular lattice structure 144, solid keel 130, and pegs 160 are built upon a forged or wrought and machined base plate 112. Regardless of manufacturing method, the cementless tibial tray implant 110 provides for an implant which may be inserted without cement, thereby reducing procedural time, cement related complications, and reducing surgeon stress.

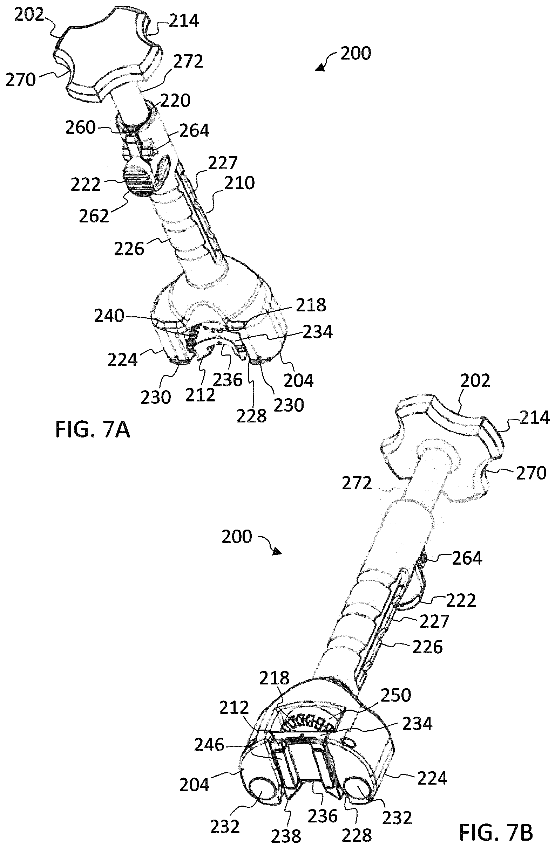

Turning now to FIGS. 7A-9B, an instrument 200 for installing a tibial implant, such as tibial tray implant 110, is shown according to one embodiment. The instrument 200 may be used to connect to tibia trays 110 of various sizes and allows for the implantation of the tibia tray 110 into a patient. After impaction and implantation, the instrument 200 may be subsequently disengaged from the tibia tray 110. The instrument 200 may be used by a single operator to aid with the implantation of the tibia tray 110 into the cavity within the tibial soft tissue, thereby allowing for quick lock and release of the tibia tray 110.

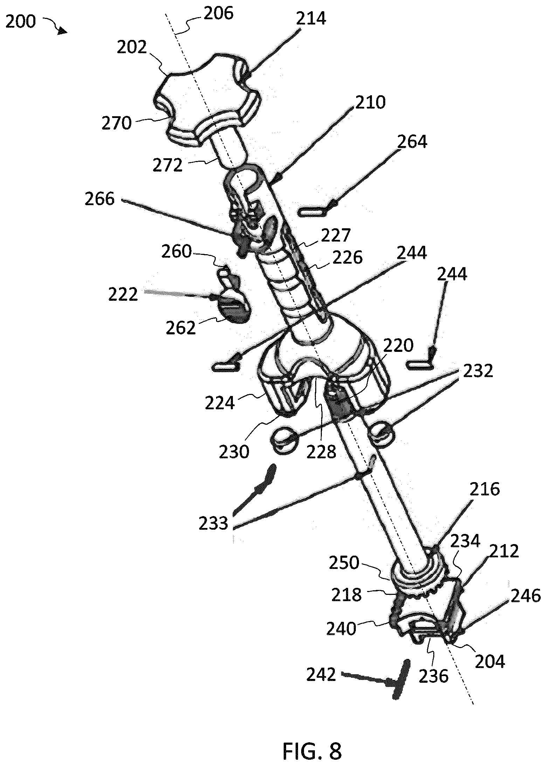

The inserter instrument 10 extends from a proximal end 202 to a distal end 204 along a central longitudinal tool axis 206. The instrument 200 includes a central or main body 210, an extendable anterior locking mechanism 212 at the distal end 204, and an impaction cap 214 at the proximal end 202. The main body 210 houses a central shaft 216 for controlling movement of the anterior locking mechanism 212. The central shaft 216 incorporates a distal gear face 218, which cooperates with the anterior locking mechanism 212 and proximal ratchet teeth 220 which cooperate with a lever mechanism 222. As best seen in the exploded view of FIG. 8, the components may be welded and pinned together to produce the assembled instrument 200.

The central or main body 210 is a hollow member with a base or foot 224 and a neck 226 protruding upwardly or proximally from the foot 224. The neck 226 is a hollow tube sized and dimensioned to receive the central shaft 216 therethrough. The neck 226 and central through opening may be generally aligned along the central longitudinal axis 206 of the instrument 200. The neck 226 may define a handle portion, for example, with grooves to be gripped by the user. The neck 226 may also define an elongated vertical window 227, which may help with cleaning or to visualize the central shaft 216.

The base or foot 224 is configured to engage with the tibial tray 110 to thereby control movement of the tibial tray 110 during impaction and insertion. The foot 224 is generally sized and shaped to fit within the insert receiving space 120 in the tibial tray 110. In other words, the outer wall of the foot 224 may have the same kidney-bean shape, which generally corresponds to the indentation 120 in the tray 110. The foot 224 may be bifurcated by a keyway 228 configured to receive the anterior locking mechanism 212. The foot 224 may include one or more posterior tabs 230 positioned along the posterior portion of the foot 224. The posterior tabs 230 may be fixed to the foot 224. The fixed posterior tabs 230 may be located on the two lobes separated by the keyway 228. The posterior tabs 230 may each be elongated with a length extending in a direction that is generally parallel to a coronal plane of the patient (e.g., a vertical side-to-side extending plane). The fixed posterior tabs 230 are receivable in the corresponding posterior pockets or notches 128 in the tibial tray 110. The bottom of the foot 224 may include one or more protectors 232, such as plastic cushions, to prevent metal on metal contact between the foot 224 and the implant 110. The protectors 232 may be secured to the bottom of the foot 224 with respective dowel pins 233, for example.

The foot 224 mimics the geometry of tibia trays 110 and houses the extendable anterior locking mechanism 212, which is configured to interface with the anterior end 124 of the tibia tray 110. The extendable anterior locking mechanism 212 includes a sliding rack 234 configured to slide in the anterior direction when actuated by the central shaft 216. The sliding rack 234 includes a body with a rectangular form having two parallel straight legs and a horizontal top, which resembles the letter U when viewed from the side. The distal end of the sliding rack 234 defines a groove 236 sized and dimensioned to interface with the center strut 122 of the tibial tray 110. The anterior portion of the sliding rack 234 includes one or more projections or tabs 238 receivable in the corresponding anterior pocket or notch 128 in the tibial tray 110. When the sliding rack 234 is translated anteriorly, the anterior tabs 238 engage notch 128, thereby securing the instrument 200 to the implant 110. The top of the sliding rack 234 defines a linear gear track 240, which when actuated by the gear face 218 on the central shaft 216, causes the sliding rack 234 to translate into or out of engagement with the tibial tray implant 110. The linear gear track 240 may define a plurality of teeth along a straight bar, which are configured to mate with corresponding teeth on the gear face 218 of the central shaft 216. The sliding rack 234 may be held in place by an anterior dowel pin 242 and two retaining pins 244, for example.

The central shaft 216 includes a cylindrical component for transmitting rotational force to the sliding rack 234. The central shaft 216 includes a gear wheel 250 with gear face 218 at the distal end, which includes a plurality of teeth that project at right angles to the plane of the gear wheel 250. The gear face 218 may be a type of bevel gear or crown gear, which is configured to mesh with the linear gear track 240. When rotational motion is applied to the central shaft 216, the intermeshing teeth of the gear face 218 and linear gear track 240 convert rotary motion into linear motion, thereby translating sliding rack 234 to the desired position. The central shaft 216 includes ratchet teeth 220 at the proximal end. The ratchet teeth 220 engage with lever 222 to rotate and lock the central shaft 216 at discrete increments. In this manner, the sliding rack 234 may be extended in the anterior direction to discrete sizes or increments. In one embodiment, the sliding rack 234 includes a corresponding protector 246, such as a plastic cushion, to prevent metal on metal wear as the sliding rack 234 slides over the center strut 122 of the implant 110.

The ratchet lever 222 includes a finger with a pawl 260 configured to engage the ratchet teeth 220 of the central shaft 216 and a button 262 for operating the ratchet lever 222. The ratchet lever 222 is secured to the main body 210 with a dowel pin 264, which the ratchet lever 222 pivots about. For example, a recess may be provided in the main body 210 with two protruding pin holes for receiving pin 264, which allows for the ratchet lever 222 to be pinned in place to interact with the central shaft 216. A spring 266 provides a tension force pushing the pawl 260 of the ratchet lever 222 against the ratchet teeth 220 of the central shaft 216 to maintain engagement. When the button 262 is depressed, the pawl 260 disengages from the ratchet teeth 220, thereby allowing for free movement of the central shaft 216. When the pawl 260 is engaged, rotation of shaft 216 allows for incremental movement of the sliding rack 234.

The impaction cap 214 includes an impaction handle 270 and a distally protruding shaft 272. The distal end of the shaft 272 mates with the proximal end of the central shaft 216. The top of the central shaft 216 may define a rectangular protrusion, for example, configured to allow the impaction cap 214 to be easily welded onto the central shaft 216. The impaction handle 270 may be grippable and controllable by a user to rotate the central shaft 216. The impaction handle 270 may include fillets within its geometry to allow for the instrument 200 to be easily rotated. The impaction handle 270 also provides a large proximal impaction face to absorb the force or impact of a hammer or mallet.

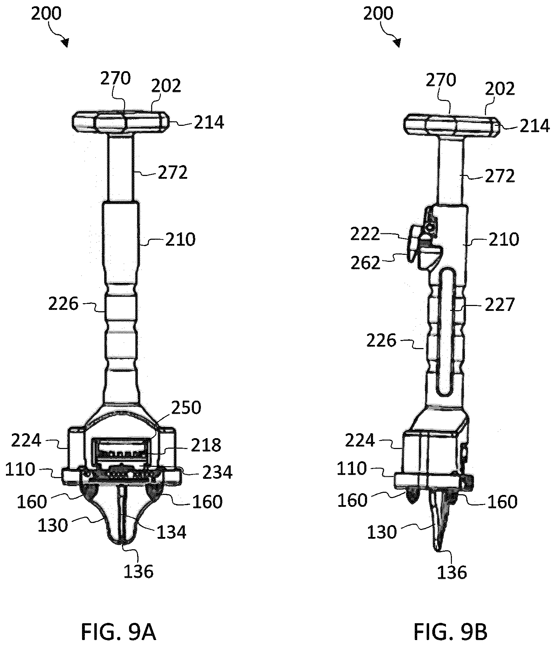

As best seen in FIGS. 9A-9B, the instrument 200 may be operated as follows. The interfacing end of the foot 224 may be positioned into the proximal face 114 of a tibia tray 110. The operator may position the lower end of the instrument 200 on top of the cementless tibia tray 110, lining up the central island 122 and the posterior notches 128 of the tray 110 with the foot 224 of the inserter device 200. Next, the operator may hold onto the central body 226 and rotate the impaction cap 202, for example, clockwise. The impaction cap 214 may be turned to rotate the central shaft 216, engaging the gear 218 with the gear rack 240 on the anterior locking mechanism 212, and extending the sliding rack 234 into the anterior groove 128 within the tibia tray 110. The instrument 200 is configured to be placed on a variety of tibia trays 110 and extended at discrete increments until the sliding rack 234 mates with the tibia tray 110. The extension may be locked at discrete distances by the ratchet teeth 220 at the top of the central shaft 216 and engagement by the pawl 260 of the lever 222. Once the sliding rack 234 has extended to as far as the tibia tray 110 will permit, the instrument 200 is in a fully locked state and retained by the ratchet interface 220, 260.

The entire instrument and tibia tray system can then be inserted, tibia tray first, into a patient's soft tissue. During insertion, the operator may impact the impaction cap 214 with a hammer, specific impactor, or a similar device until the tibia tray 110 is in the desired position. The instrument 200 is configured to hold the tibia tray 110 firmly in place while the entire system is impacted into the tibia of the patient, the keel 130 and pegs 160 are fully seated in the bone, and the trabecular surface 144 is pressed against the resected tibia.

The instrument 200 may then be easily released from the tibia tray 110. By pressing in on the ratchet lever 222, the instrument 200 unlocks, allowing the impaction cap 214 to be turned counter clockwise, for example, releasing the inserter 200 from the tibia tray 110, and allowing it to be removed without disturbing the implant 110. By pressing in the lever trigger 262 and turning the impaction cap 214 in the opposite direction, the locking mechanism 212 is collapsed, withdrawing the sliding rack 234 and leaving the tibia tray 110 behind. The instrument 200 is able to quickly lock and release tibia trays 110 without the use of hand-tightened features. Additionally, the instrument 200 is configured to interface with implants at specific ranges of extension, which improves ease of use.

Although the invention has been described in detail and with reference to specific embodiments, it will be apparent to one skilled in the art that various changes and modifications can be made without departing from the spirit and scope of the invention.

Thus, it is intended that the invention covers the modifications and variations of this invention provided they come within the scope of the appended claims and their equivalents. It is expressly intended, for example, that all components of the various devices disclosed above may be combined or modified in any suitable configuration.

The invention could be defined inter alia by the following Examples:
1. A knee arthroplasty implant system comprising: a femoral implant having an anterior flange, a pair of posterior condylar flanges, and a distal portion therebetween, the femoral implant has an outer articulating surface with a smooth rounded shape that closely approximates an exterior distal femur surface of a natural human knee and an inner surface shaped to match a resected femur with five resection cuts, and one or more pegs extending from the inner surface; and a tibial tray implant having a plate with a perimeter wall defining an insert receiving space, a distal surface configured to contact a resected tibia, a keel extending from the distal surface, and one or more pegs extending from the distal surface, wherein the inner surface of the femoral implant and the distal surface of the tibial tray define a trabecular structure, which provides a scaffold for bone healing and interdigitation.
2. The system of claim 1, wherein the trabecular structure has a grid, lattice, or honeycomb pattern to promote bony in-growth.
3. The system of Example 1, wherein the trabecular structure has a porosity in the range of 50-80%.
4. The system of Example 1, wherein a thickness of the trabecular structure is oversized for an increased press fit in trabecular structure areas to ensure contact with the resected femur and tibia.
5. The system of Example 1, wherein the trabecular structure is created by three-dimensional printing.
6. The system of Example 1, wherein the pegs of the femoral implant and tibial tray implant each have a bullet shaped profile with six fins radiating outward from the peg.
7. The system of Example 1, wherein the inner surface of the femoral implant includes an anterior femur cut surface, a posterior femur cut surface, a distal femur cut surface, an anterior chamfer cut surface, and a posterior chamfer cut surface.
8. The system of Example 7, wherein the trabecular structure extends along the anterior femur cut surface, the posterior femur cut surface, the distal femur cut surface, the anterior chamfer cut surface, and the posterior chamfer cut surface.
9. The system of Example 1, wherein the trabecular structure is surrounded by a solid wall.
10. The system of Example 1, wherein the femoral implant and tibial implant are cementless and are securable to bone without cement.
11. A system for a knee arthroplasty, the system comprising:
   a tibial tray having a perimeter wall defining an insert receiving space, the perimeter wall defining a pair of posterior notches and a single anterior notch; and
   an inserter having a main body with a foot having a pair of fixed posterior tabs, a sliding rack having an anterior tab, and a rotatable shaft for controlling movement of the sliding rack, wherein the posterior tabs are receivable in the posterior notches of the tibial tray and the anterior tab is receivable in the anterior notch of the tibial tray when the shaft is rotated and the sliding rack is translated into an extended position.
12. The system of Example 11, wherein the rotatable shaft has a gear wheel with a gear face at its distal end.
13. The system of Example 12, wherein the sliding rack has a linear gear track with teeth that intermesh with the gear face of the rotatable shaft.
14. The system of Example 11, wherein the foot is bifurcated by a keyway, and the sliding rack is receivable in the keyway.
15. The system of Example 11, wherein the tibial tray has a kidney-bean shape with an anterior side forming an outer convex side and a posterior side including an inner concave side separating two lobes, and the foot has an outer kidney-bean shape matching the kidney-bean shape of the tibial tray.
16. The system of Example 11, wherein the tibial tray includes a keel attached to a distal surface of the tibial tray, and the keel includes a pair of coronal fins and a sagittal fin.
17. A method for implanting a tibial implant, the method comprising:
   positioning fixed posterior tabs on a main body of an inserter into corresponding posterior notches in a perimeter wall of a tibial tray;
   tilting the inserter until a foot of the main body of the inserter is received in an insert receiving space of the tibial tray;
   rotating a shaft through the main body of the inserter to translate a sliding rack having an anterior tab and extend the anterior tab into an anterior notch in the tibial tray, thereby locking the inserter to the tibial tray; and
   impacting an impaction cap to seat the tibial tray into a proximal tibia of a patient.
18. The method of Example 17 further comprising, before positioning the inserter, resecting the proximal tibial to form a planar resection surface.
19. The method of Example 17 further comprising, before positioning the inserter, punching a cavity into the proximal tibia, the cavity being configured to receive a keel of the tibial tray.
20. The method of Example 17 further comprising removing the inserter by rotating the shaft in an opposite direction and withdrawing the sliding rack from the tibial tray, and attaching an insert with tabs receivable in the posterior and anterior notches in the tibial tray.

## Claims

1. A knee arthroplasty implant system comprising:
a femoral implant having an anterior flange, a pair of posterior condylar flanges, and a distal portion therebetween, the femoral implant has an outer articulating surface with a smooth rounded shape configured to closely approximates an exterior distal femur surface of a natural human knee and an inner surface shaped to match a resected femur with five resection cuts, and one or more pegs extending from the inner surface; and
a tibial tray implant having a plate with a perimeter wall defining an insert receiving space, a distal surface configured to contact a resected tibia, a keel extending from the distal surface, and one or more pegs extending from the distal surface,
wherein the inner surface of the femoral implant and the distal surface of the tibial tray define a trabecular structure, which provides a scaffold for bone healing and interdigitation.

2. The system of claim 1, wherein the trabecular structure has a grid, lattice, or honeycomb pattern to promote bony in-growth.

3. The system of claim 1 or 2, wherein the trabecular structure has a porosity in the range of 50-80%.

4. The system of any one of the preceding claims, wherein a thickness of the trabecular structure is oversized for an increased press fit in trabecular structure areas to ensure contact with the resected femur and tibia.

5. The system of any one of the preceding claims, wherein the trabecular structure is created by three-dimensional printing.

6. The system of any one of the preceding claims, wherein the pegs of the femoral implant and tibial tray implant each have a bullet shaped profile with six fins radiating outward from the peg.

7. The system of any one of the preceding claims, wherein the inner surface of the femoral implant includes an anterior femur cut surface, a posterior femur cut surface, a distal femur cut surface, an anterior chamfer cut surface, and a posterior chamfer cut surface.

8. The system of claim 7, wherein the trabecular structure extends along the anterior femur cut surface, the posterior femur cut surface, the distal femur cut surface, the anterior chamfer cut surface, and the posterior chamfer cut surface.

9. The system of any one of the preceding claims, wherein the trabecular structure is surrounded by a solid wall.

10. The system of any one of the preceding claims, wherein the femoral implant and tibial implant are cementless and are securable to bone without cement.

11. A system for a knee arthroplasty, the system comprising:
a knee arthroplasty implant system according to any one of the preceding claims, wherein the perimeter wall of the tibial tray defines a pair of posterior notches and a single anterior notch; and
an inserter having a main body with a foot having a pair of fixed posterior tabs, a sliding rack having an anterior tab, and a rotatable shaft for controlling movement of the sliding rack, wherein the posterior tabs are receivable in the posterior notches of the tibial tray and the anterior tab is receivable in the anterior notch of the tibial tray when the shaft is rotated and the sliding rack is translated into an extended position.

12. The system of claim 11, wherein the rotatable shaft has a gear wheel with a gear face at its distal end.

13. The system of claim 11 or 12, wherein the sliding rack has a linear gear track with teeth that intermesh with the gear face of the rotatable shaft.

14. The system of any one of claims 11 to 13, wherein the foot is bifurcated by a keyway, and the sliding rack is receivable in the keyway.

15. The system of any one of claims 11 to 14, wherein the tibial tray has a kidney-bean shape with an anterior side forming an outer convex side and a posterior side including an inner concave side separating two lobes, and the foot has an outer kidney-bean shape matching the kidney-bean shape of the tibial tray and wherein preferably the tibial tray includes a keel attached to a distal surface of the tibial tray, and the keel includes a pair of coronal fins and a sagittal fin.
